# EUROPEAN PATENT APPLICATION

(11) **EP 3 970 791 A1**
(43) Date of publication of application: **23.03.2022**
(21) Application number: 20197105.8
(22) Date of filing: 21.09.2020
(51) Int. Cl.: A61N 7/00, A61C 19/04

(54) **SYSTEM FOR PROVIDING ORAL TISSUE TREATMENT**

(71) Applicant: Koninklijke Philips N.V., 5656 AG Eindhoven (NL)
(72) Inventor: PEKAR, Martin, 5656 AE Eindhoven (NL); RMAILE, Amir Hussein, 5656 AE Eindhoven (NL); GOTTENBOS, Bart, 5656 AE Eindhoven (NL); PRESURA, Cristian Nicolae, 5656 AE Eindhoven (NL)
(74) Representative: Philips Intellectual Property & Standards

(57) **Abstract**

A system for providing oral tissue treatment has an ultrasound patch for application externally to the face of a user and comprising an array of ultrasound transducers. The patch is used in an imaging mode to capture the oral geometry of a user of the system and the treatment location or locations are identified from the image. The patch is also used in a treatment mode to deliver ultrasound energy selectively to the identified treatment location or locations.

## Description

### FIELD OF THE INVENTION

This invention relates to the treatment of oral tissue, in particular using ultrasound.

### BACKGROUND OF THE INVENTION

Low intensity pulsed ultrasound (LIPUS) is known to have a benefit on bone fracture healing. Mechanical nano-motion at the fracture site caused by LIPUS triggers a chain of biochemical reactions, of which the clinical effect is increased expression of osteogenic genes and thus enhanced re-mineralization of the bone.

In the dental field, a LIPUS product, the intraoral Aevo (TM) system known as SmileSonica (TM), is known for accelerating orthodontic tooth movement, reducing the needed time to wear braces. LIPUS has also been proposed to possibly benefit periodontitis or gingivitis patients, as it may reduce inflammation and may strengthen or heal periodontal tissues.

LIPUS treatments typically become effective when used daily used for extended periods. The intraoral Aevo (TM) system is in the form of a mouthpiece which is worn by the user. The mouthpiece needs to be used daily typically for 20 minutes for a total period of about a year. Compliance is a big issue because of this. Indeed, clinical studies have shown that only 1 out of 4 users are compliant (compliance being defined as the device being used for more than two thirds of the desired time). Wearing an intraoral mouthpiece for 20 minutes each day is obviously very inconvenient for the user.

There is a need for an oral treatment system based on ultrasound which is more convenient for a user.

### SUMMARY OF THE INVENTION

The invention is defined by the claims.

According to examples in accordance with an aspect of the invention, there is provided a system for providing oral tissue treatment, comprising:
an ultrasound patch for application externally to the face of a user and comprising an array of ultrasound transducers; and
a controller for controlling the ultrasound transducer array of the ultrasound patch,
wherein the controller is adapted to control the ultrasound transducer array in an imaging mode and in a treatment mode, wherein:
   in the imaging mode, an image is acquired capturing the oral geometry of a user of the system, and the controller is adapted to identify a treatment location or locations from the image; and
   in the treatment mode, the controller is adapted to control the ultrasound transducer array to deliver ultrasound energy selectively to the identified treatment location or locations.

The system is for example for promoting bone healing or for providing tissue treatment, such as for the treatment of gingiva.

The system enables targeted delivery of ultrasound treatment energy by using an ultrasound patch to locate the treatment location based on the imaging capability of the ultrasound transducer patch. The treatment capability of the ultrasound probe is then used in the treatment mode. The patch is applied externally to the face (e.g. to the cheek) so provides a convenient system for the user.

The controller may be attached to the patch but it may be on a remote device such as a mobile phone or tablet.

The ultrasound transducer array for example comprises an array of CMUT transducer elements. This provides a low cost, efficient, highly-integrable and flexible ultrasound solution. Other technologies may however be used such as piezoelectric transducers.

The CMUT transducer elements may be operated in the collapse mode during the treatment mode. This provides a best frequency and pressure response of the ultrasound transducer elements for the desired treatment output.

The ultrasound patch for example comprises a flexible printed circuit board carrying the ultrasound transducer array.

The system may comprise a multiplexer for multiplexing signals between the controller and the ultrasound patch.

This reduces the amount of wiring needed. The multiplexing is for example based on the use of the bias voltage of CMUT transducer elements. Thus, a sub-array may be activated which shares bias voltage terminals, and the individual transducer elements within that sub-array may then be activated based on control signals.

The treatment location or locations for example comprise regions of dental bone tissue.

The ultrasound energy used in the treatment mode for example has a frequency in the range 1MHz to 3MHz. The frequency used for imaging may be the same as for treatment, or might it be different. If different frequencies are used, a frequency sweep may be used during treatment to avoid hotspots. In this way, a longer treatment time per session may be allowed.

The ultrasound energy used in the imaging mode for example has a frequency in the range 3MHz to 10MHz.

Frequency switching between the treatment mode and the imaging mode, if required, may be implement either by utilizing bias voltage-enabled frequency tunability of CMUT ultrasound transducer elements, or by utilizing the bandwidth of a PZT transducer.

Frequency selection may also be used for penetration depth control (e.g. the ultrasound energy needs to penetrate deeper in the case of obese people with a thick cheek). CMUT devices for example enable the required flexibility in terms of switching between frequencies.

In the treatment mode, the transducer array is for example adapted to provide pulsed ultrasound energy. This has been found to provide optimum treatment results.

The pulsed ultrasound energy for example has one or more of:
a pulse repetition frequency in the range 50Hz to 2kHz;
a number of pulses per treatment session in the range 100 to 10,000;
a duty cycle in the range 5% to 50%; and
a time averaged delivered intensity in the range 10mW/cm² to 500mW/cm².

The controller may be adapted to identify a treatment location or locations from the image by:
identifying landmarks within the image; and
interpolating a treatment location from the landmarks.

The landmarks are for example the teeth or implant structures such as braces, and the treatment location is for example a tooth root.

The controller may be adapted to identify a treatment location or locations in real time and the treatment mode is adapted in real time.

The system may for example operate in the imaging mode and the treatment mode alternately. The imaging mode provides information to the treatment mode about the treatment location. This can happen very fast with relative to a relatively slow movement, such as movement of the mouth. For example, movement of the user caused by talking may be tracked to maintain the effective treatment at the intended location.

The controller may be adapted to control the ultrasound transducer array to deliver ultrasound energy selectively to the identified treatment location or locations by one or more of:
angular repositioning of the ultrasound probe;
implementing bi-plane imaging;
activating different parts of the ultrasound array.

Thus, there are various ways to provide targeted treatment.

The invention also provides a computer program comprising computer program code which is adapted, when run on the processor of a controller of a system for providing oral tissue treatment, to implement a method comprising:
in an imaging mode:
   controlling an array of ultrasound transducers of an ultrasound patch to acquire an image capturing the oral geometry of a user of the system; and
   identifying a treatment location or locations from the image; and
in a treatment mode:
   controlling the ultrasound transducer array to deliver ultrasound energy selectively to the identified treatment location or locations.

The method for example comprises, in the treatment mode, providing pulsed ultrasound energy, wherein the pulsed ultrasound energy has one or more of:
a pulse repetition frequency in the range 50Hz to 2kHz;
a number of pulses per treatment session in the range 100 to 10,000;
a duty cycle in the range 5% to 50%;
a time averaged intensity in the range 10mW/cm² to 500mW/cm².

The invention also provides a processor having a memory on which is stored the computer program defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for providing oral tissue treatment;
Figure 2 schematically depicts a typical CMUT element of an ultrasound system operable in a collapsed mode;
Figures 3 and 4 depict operating principles of such a CMUT element;
Figure 5 shows the image analysis process;
Figure 6 shows the ultrasound patch and the captured image, to explain the image processing.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for providing oral tissue treatment, having an ultrasound patch for application externally to the face of a user and comprising an array of ultrasound transducers. The patch is used in an imaging mode to capture the oral geometry of a user of the system and the treatment location or locations are identified from the image. The patch is also used in a treatment mode to deliver ultrasound energy selectively to the identified treatment location or locations.

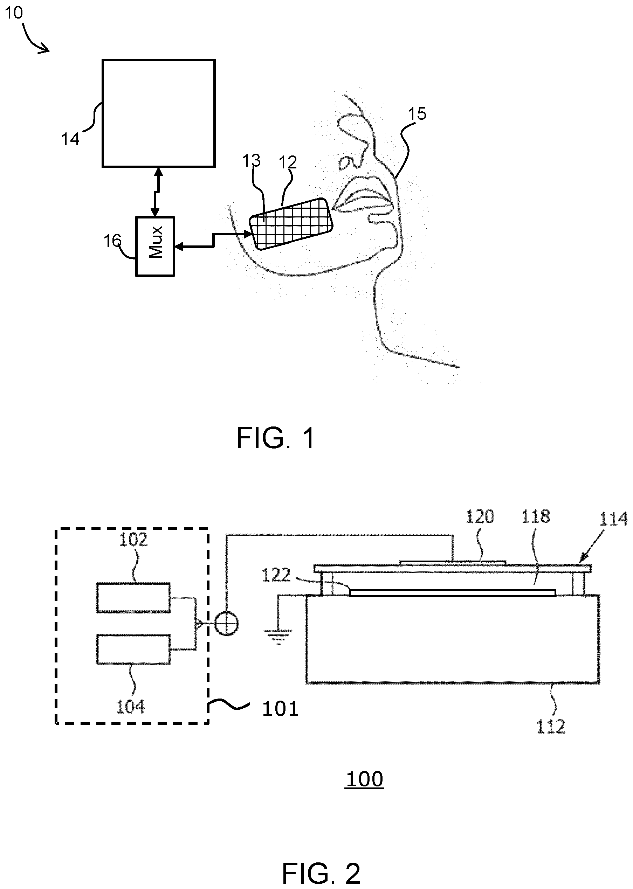

Figure 1 shows a system 10 for providing oral tissue treatment. It comprises the ultrasound patch 12, comprising an array of ultrasound transducers 13, for application externally to the face of a user 15. A controller 14 controls the ultrasound transducer array of the ultrasound patch in different modes.

In particular, there is an imaging mode in which an image is acquired capturing the oral geometry of the user 15. The controller 14 then identifies a treatment location or locations from the image.

There is also a treatment mode in which the controller 14 controls the ultrasound transducer array to deliver ultrasound energy selectively to the identified treatment location or locations.

The system is for example for promoting bone healing or for providing tissue treatment, such as for the treatment of gingiva.

The system enables targeted delivery of ultrasound treatment energy by using the ultrasound patch to locate the treatment location based on ultrasound image analysis. The treatment capability of the ultrasound probe is then used in the treatment mode. The patch is applied externally to the face (e.g. to the cheek) so provides a convenient system for the user.

In the example shown, the controller is remote from the patch 12, for example implemented by a mobile device of the user with wireless transfer of control data to the patch. The patch for example has a battery for powering the ultrasound transducers and for data communications. The controller may instead be integrated into the patch. The ultrasound patch for example comprises a flexible printed circuit board carrying the ultrasound transducer array, so that the patch may be fitted to the shape of the face of the user.

Figure 1 also shows a multiplexer 16 for multiplexing signals between the controller 14 and the ultrasound patch 12. This reduces the amount of wiring needed for the imaging mode. The multiplexing is for example based on use of the bias voltage of CMUT transducer elements, as discussed in more detail below.

The multiplexing is used in the imaging mode, and for switching between the imaging and treatment modes, for example if different ultrasound array elements are needed for the imaging and for the treatment. The multiplexing is not needed for the treatment mode.

The patch 12 may comprise strips for the upper and the lower jaw, attached with an ultrasound gel to guide the ultrasound into the cheeks. Alternatively, patch strips may be strapped to the head in a similar manner to a (e.g. respiratory) mask. This may allow more freedom of movement, and less or even no need for coupling gel.

In a more advanced solution, the design may even be customized, to fit the shape of the face. In this case, the positioning may be more reproducible, and the directivity of the ultrasound may then be designed to treat the most important parts of the periodontium taking into account the known way the patch is worn.

Typically, the treatment may be used for the full dentition at once, since treatment times are of the order of tens of minutes. The patch is therefore preferably formed as extra oral flexible ultrasound patch attached to the cheek and extending over the full dentition area as shown in Figure 1. There may be separate patches of the type shown on each side of the face, or else the treatment may be limited to an area of the mouth in which case a single patch may suffice.

Only the outside of the periodontium will be treated by an external patch, but for example for orthodontics this is sufficient, and indeed the Aevo (TM) system referenced above only treats from the outside.

The typical parameters used in LIPUS are a 1.5MHz ultrasound frequency, using pulse bursts of 300 pulses, delivered during 0.2ms, at a pulse-repetition frequency of 1kHz (20% duty cycle) and a 30mW/cm² time averaged intensity.

More generally, the pulsed ultrasound energy for example has one or more of:
a frequency in the range 1MHz to 3MHz;
a pulse repetition frequency in the range 50Hz to 2kHz;
a number of pulses per treatment session in the range 100 to 10,000;
a duty cycle in the range 5% to 50%; and
a time averaged delivered intensity in the range 10mW/cm² to 500mW/cm².

On bone cells, this type of treatment has been shown to increase the production of nitric oxide and certain prostaglandins, which stimulate the production of new bone. Clinically, a faster healing of bone fractures has been proven, and there are promising initial clinical studies that also orthodontic tooth movement can be achieved using these ultrasound characteristics.

Studies also show a faster healing of oral surgery, such as dental implantation, under influence of LIPUS. It has been proposed that periodontal bone damaged due to periodontitis may also benefit from LIPUS, and that LIPUS even may have antiinflammatory effects.

The frequency range around 1.5 MHz is found to be optimal for LIPUS, which is explained by the fact that the ultrasound produces nano-motion at the treatment site, which triggers the biological response. Frequencies below 1MHz have been found to be less effective, likely because the relevant structures are not as easily vibrated using lower frequencies.

The frequency used for imaging may be the same as for treatment, or might it be different. If different frequencies are used, a frequency sweep may be used during treatment to avoid hotspots. In this way a longer treatment time per session may be allowed.

The ultrasound energy used in the imaging mode for example has a frequency in the range 3MHz to 10MHz. Frequency switching between the treatment mode and the imaging mode, if required, may be implement either by utilizing bias voltage-enabled frequency tunability of CMUT ultrasound transducer elements, or by utilizing the bandwidth of a PZT transducer.

The preferred technology for manufacturing the flexible ultrasound transducer patches is the collapse-mode capacitive-micromachined ultrasonic transducer (CMUT) technology.

Figure 2 shows a known design of CMUT element 100 (a so-called cell) for use in an ultrasound system and a known drive arrangement 101. The CMUT element 100 comprises a flexible membrane or diaphragm 114 suspended above a silicon substrate 112 with a gap or cavity 118 there between. A first electrode 122 is located on the floor of the drum on the upper surface of the substrate 112 in this example. A second electrode 120 is located on the diaphragm 114 and moves with the diaphragm. In the example shown, the two electrodes are circular.

A dielectric (not shown) is provided on the substrate 112 and underneath the top (second) electrode 120. The diaphragm may instead function as a dielectric layer.

Preferably, there are two dielectrics which may be equal in composition and thickness, but may be also asymmetric (different materials and thicknesses).

The membrane layer 114 is fixed relative to the top face of the substrate layer 112 and configured and dimensioned so as to define a spherical or cylindrical cavity 118 between the membrane layer 114 and the substrate layer 112.

Other realizations of the electrode 120 design can be considered, such as electrode 120 may be embedded in the membrane 114 or it may be deposited on the membrane 114 as an additional layer.

The first electrode may be directly exposed to the gap 118 or separated from the gap 118 by an electrically insulating layer or film to prevent a short-circuit between the second electrode 120 and the first electrode 122.

In Figure 2 the first electrode 122 is grounded by way of example. Other arrangements, e.g. a grounded second electrode 120 or both second electrode 120 and first electrode 122 floating are of course equally feasible.

The electrodes of the CMUT element 100 provide the capacitive plates of the device and the gap 118 is the main dielectric between the plates of the capacitor. When the diaphragm vibrates, the changing dimension of the dielectric gap between the plates provides a changing capacitance which is sensed as the response of the CMUT element 100 to a received acoustic echo.

The spacing between the electrodes is controlled by applying a static voltage, e.g. a DC bias voltage, to the electrodes with a voltage supply 101. The voltage supply 101 may optionally comprise separate stages 102, 104 for providing the DC and AC or stimulus components respectively of the drive voltage of the CMUT elements 100, e.g. in transmission mode. The first stage 102 may be adapted to generate the static (DC) voltage component and the second stage 104 may be adapted to generate an alternating variable drive or stimulus voltage component having a set alternating frequency, which signal typically is the difference between the overall drive voltage and the aforementioned static component thereof.

The static or bias component of the applied drive voltage preferably meets or exceeds the threshold voltage for forcing the CMUT element 100 into its collapsed state. This has the advantage that the first stage 102 may include relatively large capacitors, e.g. smoothing capacitors, in order to generate a particularly low-noise static component of the overall voltage, which static component typically dominates the overall voltage such that the noise characteristics of the overall voltage signal will be dominated by the noise characteristics of this static component.

It is known that by applying a static voltage above a certain threshold, the CMUT element 100 is forced into a collapsed state in which the membrane 114 collapses onto the substrate 112. This threshold value may depend on the exact design of the CMUT element 100 and is defined as the DC bias voltage, known as the collapse voltage, at which the membrane 114 sticks to (contacts) the drum floor through the force due to the electric field between the electrodes. The amount (area) of contact between the membrane 114 and the substrate 112 is dependent on the applied bias voltage.

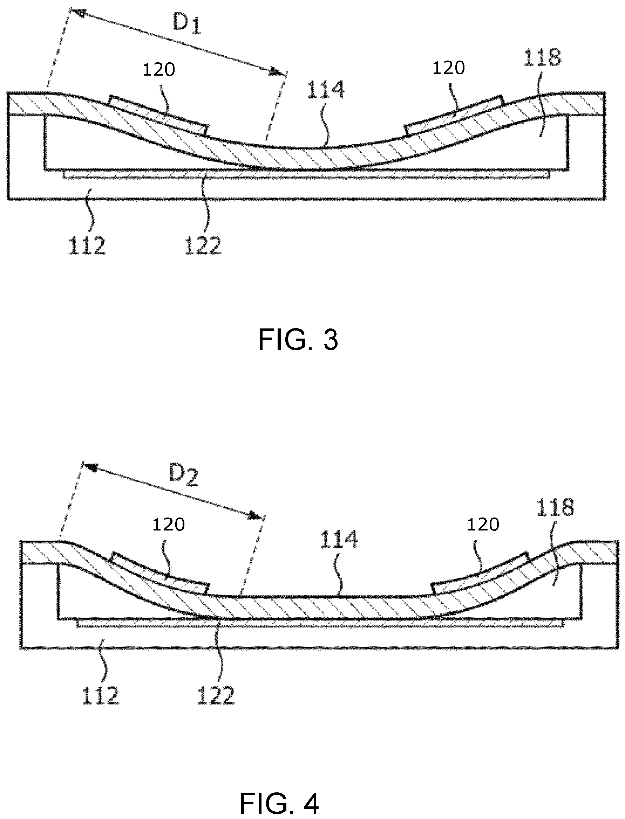

Increasing the contact area between the membrane 114 and the substrate 112 increases the resonant frequency of the membrane 114, as will be explained in more detail with the aid of Figure 3 and Figure 4.

The frequency response of a collapsed mode CMUT element 100 may be varied by adjusting the DC bias voltage applied to the CMUT electrodes after collapse. As a result, the resonant frequency of the CMUT element increases as a higher DC bias voltage is applied to the electrodes.

The principles behind this phenomenon are illustrated in Figures 3 and 4. The cross-sectional views of Figures 3 and 4 illustrate this one-dimensionally by the distances D1 and D2 between the outer support of the membrane 114 and the point where the membrane begins to touch the floor of the cavity 118 in each illustration. It can be seen that the distance D1 is a relatively long distance in Figure 3 when a relatively low bias voltage is applied, whereas the distance D2 in Figure 4 is a much shorter distance due to a higher bias voltage being applied. These distances can be compared to long and short strings which are held by the ends and then plucked. The long, relaxed string will vibrate at a much lower frequency when plucked than will the shorter, tighter string. Analogously, the resonant frequency of the CMUT element in Figure 3 will be lower than the resonant frequency of the CMUT element in Figure 4 which is subject to the higher bias voltage.

Thus, a typical CMUT design comprises a flexible membrane or diaphragm, for example formed of silicon nitride, suspended above a silicon substrate with a gap or cavity between them. The gap is caused by removing a sacrificial layer during manufacture. A first electrode is located on the floor of the drum on the upper surface of the substrate and a second electrode is located on the diaphragm and moves with the diaphragm.

The radius of a single CMUT membrane for example ranges from 10 to 200µm. The cavity height is a few hundred nanometres and the total height of a CMUT element is a few µm. Multiple CMUT cells, of which the top and bottom electrodes are shortcircuited, respectively, can be arranged in an array resulting in a large active area. The bias voltage can then be used as an on/off switch for particular segments providing a simple multiplexing solution, as mentioned above.

The frequency response of a CMUT is dependent on the membrane radius, height of the cavity and thickness of the top flexible membrane, as well as the static (bias) voltage as explained above. Typically, four modes of CMUT operation are distinguished: (a) conventional mode, (b) collapse mode, (c) deep-collapse mode, and (d) collapse-snapback mode. These modes differ in both frequency and pressure output.

The conventional mode offers a low frequency response as the center point of the top flexible membrane bends only by about one third of the cavity height, at which point the attractive electrostatic force equals the repulsive elastic force of the membrane. This limits the magnitude of the electrostatic forces and the maximum pressure output. The advantage of this mode is ease of manufacturing and long-term stability.

An increase in the static bias voltage increases the electrostatic forces which eventually bring the top flexible membrane in the collapse mode, with contact to the bottom electrode. The frequency of operation in the collapse mode is typically at least two times higher than the conventional mode, while the difference in acoustic pressure output is much smaller (with a higher pressure output for the collapse mode due to the larger electric field).

In the collapse mode the displacement of the membrane is limited to circumferential displacement of the top flexible membrane of which the center is in contact with the insulated bottom electrode. This mode results in a large electric field up to 100V/µm, so the pressure output is higher than that of the conventional mode.

A further increase in bias voltage increases the operating frequency as the device enters the deep-collapse mode. It has been shown that the frequency response of a CMUT operated in the deep collapse mode can be tuned from 6-18MHz. By utilizing instantaneous fractional bandwidth available at the given bias voltage (typically 40-120%), the frequency response of the CMUT can be tuned further.

The collapse-snapback mode is a highly non-linear mode having a frequency close to that of the conventional mode. This is the highest pressure mode, in which the membrane intermittently makes contact with the substrate and releases. Simulations show that the pressure output in the collapse-snapback mode is up to 22 times higher than that of the conventional mode, and thus about 17 times higher than that of the collapse mode. Also, the frequency of operation in the collapse-snapback mode is about 3 to 4 times lower than the (deep) collapse mode as the membrane exhibits displacement over the whole cavity height.

Acoustic intensities up to 6-30 W/cm² have been achieved in the collapse-snapback mode of operation at frequencies of 3-8MHz, respectively. Although these intensities have previously been used for high-intensity focused ultrasound ablations and are much higher than what is needed for LIPUS, they demonstrate the capability of the CMUT technology to generate high intensity fields.

Different modes of operation may thus be used to achieve different responses. Given the frequency and pressure responses, it is proposed to utilize the collapse mode of operation (medium pressure output, robust frequency response) for LIPUS treatment, and typically also for the ultrasound imaging function.

In addition, the bias voltage supply to the individual transducer arrays on the flexible patch may be used as multiplexers to selectively activate specific sub-arrays in the imaging mode to achieve cable reduction, as mentioned above.

Table 1 below shows example parameter windows (the column "Range") for LIPUS, and a proposed configuration (the column "LIPUS") for an extra oral ultrasound transducer patch.

**Table 1**

| **Table 1. Parameter** | **LIPUS** | **Range** |
|---|---|---|
| **Frequency** | 1.5MHz | 1-3MHz |
| **Cycles/B** | 300 | 100-10,000 |
| **Burst length** | 0.2ms | 0.1-10ms |
| **Pause time.** | 0.8ms | 0.4-90ms |
| **Burst freq.** | 1000Hz | 50-2000Hz |
| **Duty cycle** | 20% | 5-50 % |
| **US pressure** | 0.06 MPa | 0.03-0.2MPa |
| **Intensity burst** | 0.15W/cm² | 0.03-1.3W/cm² |
| **Time average I** | 0.03W/cm² | 0.01-0.5W/cm² |

The treatment location or locations to which the LIPUS treatment is to be directed are determined based on image analysis using standard image processing techniques, implemented by the controller 14.

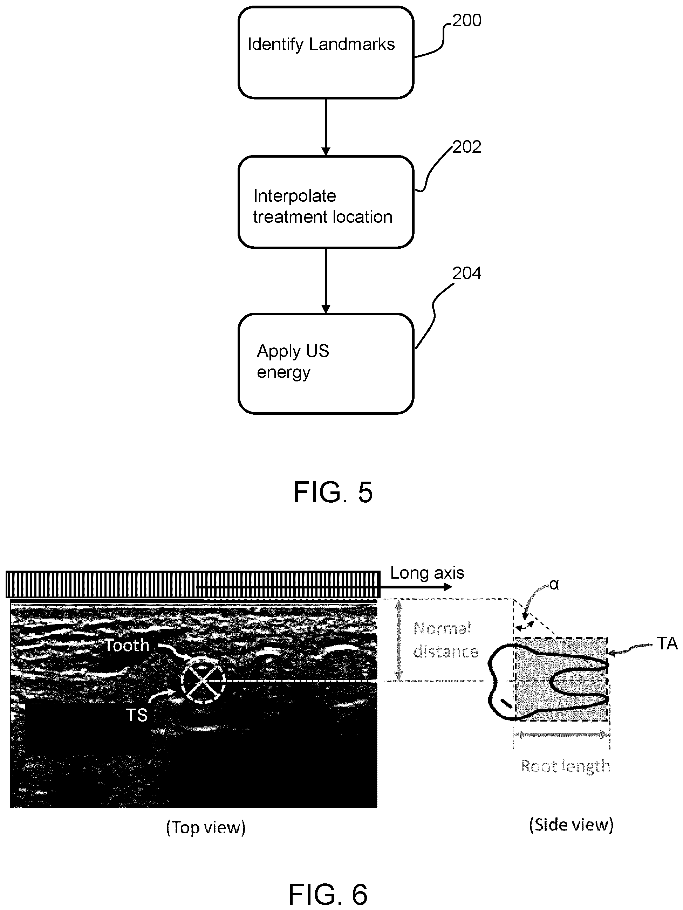

Figure 5 shows the image analysis process.

First, in step 200, landmarks are identified in the obtained ultrasound image. This landmark identification relies on existing segmentation algorithms (graph cut, convolution filters, mean shift, etc.).

Next, an interpolation algorithm is used in step 202 to identify the treatment location. For example, the geometrical center of the segmented tooth contour is detected and a beam steering angle is calculated based on the root length.

The ultrasound energy is then applied to desired treatment area in step 204. The process is repeated to continuously adapt for mouth movements.

Figure 6 shows the ultrasound patch 12 having an array of ultrasound transducer elements 13. The direction of delivery of energy is generally perpendicular to the patch (and perpendicular to the long axis of the ultrasound array). A resulting ultrasound image is shown. The teeth can be identified in the image by image analysis, to create a tooth segment TS. The individual teeth are identifiable in the ultrasound image, and the ultrasound energy for example needs to be applied to the roots of the teeth, and hence a different location to the imaged area. Since each tooth is a rigid structure and the distance between the top of the tooth to the root (the "Root length" shown in Figure 6) is known to be 1-2 cm, or can be derived more accurately from orthodontic examinations, the treatment location can be easily interpolated.

Figure 6 shows the intended treatment area TA and also shows a beam angle α to direct the treatment to the desired location (the center of the root tip). This angle α may be used as the parameter representative of the treatment location.

Note that other landmarks such as braces (typically made of metal, therefore clearly recognizable in an ultrasound image) can be used to identify and/or interpolate the treatment location.

When applying the ultrasound energy in step 204, the controller 14 can apply various techniques to implement the directional control.

A first approach is to reposition the ultrasound probe by the determined angle α. This requires some tracking mechanism in the transducer, such as accelerometers.

A second approach is to switch the ultrasound probe to apply energy in a plane perpendicular to the imaging plane, thereby implementing bi-plane imaging.

Bi-plane imaging allows the system to switch between two planes. Within each plane, ultrasound energy can be focused in the shape of a beam which is then scanned over the entire angle α of Figure 6. The focusing and scanning is implemented by means of electronic delays as is well-known to a person skilled in the art of ultrasound. The delay lines can be common to both the imaging and the treatment planes. The adjustable delays become effective either to the imaging plane or to the treatment plane, depending on which plane is switched on by the bi-plane functionality or by the bias voltage multiplexing.

A third approach is to activate different parts of the ultrasound array. The treatment area is for example located 1-2 cm (root length) in a normal direction below the activated parts of the imaging array.

The probe is positioned on the cheek. The treatment array may be activated only when the correct transducer position is registered by the imaging array. Although this is the simplest solution not requiring any accelerometers or bi-plane switching electronics, it leads to the longest treatment time.

CMUT elements are preferred for implementing multi-element flexible ultrasound patches, as is made clear above. However, more traditional ultrasound transducers may be used. Piezoelectric transducers may for example also be embedded in a flexible patch and offer the required functionality for LIPUS treatment.

The imaging mode may involve the conventional use of a CMUT (or other) transducer array) for ultrasound imaging. Typically the transducer array is a two-dimensional array of transducers capable of scanning either a 2D plane or a three dimensional volume of a region of interest.

Ultrasound imaging makes use of a beamformer system (which may be a system beamformer and optionally also a microbeamformer), which controls reception of signals by the transducer elements. A line-by-line imaging sequence is typically followed, in which the beamformer system activates the transducer array, or a sub-aperture of the transducer array. The sub-aperture may be a one dimensional line of transducers or a two dimensional patch of transducers within the larger array.

In transmit mode, the focusing and steering of the ultrasound beam generated by the array, or a sub-aperture of the array, are controlled.

Upon receiving the backscattered echo signals, the received signals undergo receive beamforming in order to align the received signals, and, in the case where a sub-aperture is being used, the sub-aperture is then shifted.

The direction in which beams are steered and focused is controlled. Beams may be steered straight ahead from (orthogonal to) the transducer array, or at different angles for a wider field of view. The steering and focusing of the transmit beam may be controlled as a function of transducer element actuation time.

The beamformed reception signals are coupled to a signal processor of the controller. The signal processor processes the received echo signals in various ways, such as: band-pass filtering; decimation; I and Q component separation; and harmonic signal separation. The processed echo signals are used to generate the ultrasound image.

Some design considerations for the patch will now be explained, simply for the purpose of clarity.

The patch preferably covers the full length of the cheek, so that is for example approximately 8 cm long (along the long axis shown in Figure 1). Tooth roots are about 1 to 2 cm long, so the patch is for example approximately 2 cm wide (along the short axis shown in Figure 1). This results in the patch area needed to treat one half of the upper or lower jaw as shown in Figure 1.

To obtain sufficient imaging aperture, the distance between the elements is preferably between λ and 2λ, where λ denotes the acoustic wavelength in water.

The image depicted in Figure 6 was obtained using a linear array transducer (the Philips L12-3 broadband linear transducer array) with a frequency of 7.5MHz, and an aperture length of 38mm, with 160 transducer elements. The acoustic wavelength is λ = 205µm (based on a speed of sound in water of 1540 m/s). The element width is 38000/160 = 240µm, hence 1.17 λ.

A lower frequency, hence with a lower resolution, may however be used, and an increased element width may also be used (which will decrease the long-axis steering capability). A 2λ element width may then provide an 8 cm long array with only 80 elements.

The number of elements in the short axis direction can be as low as 1. In such a case, no steering is possible and the treatment array is activated only when the correct transducer position is registered by the imaging array as described above.

If the beam is to be steered through the angle α as illustrated in Figure 6, the element width is then preferably in the range 1λ to 4λ. Considering the LIPUS treatment frequency of 1.5 MHz (λ = 1mm) and array height of 2 cm, a preferred number of elements along the short axis direction (i.e. the number of rows in the patch) is in the range 5 to 20.

Element widths larger than 2λ will commonly result in imaging artifact called grating lobes. This is, however, not a problem because the treatment plane is not used for imaging.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A system (10) for providing oral tissue treatment, comprising:
an ultrasound patch (12) for application externally to the face of a user and comprising an array of ultrasound transducers (13); and
a controller (14) for controlling the ultrasound transducer array of the ultrasound patch,
wherein the controller (14) is adapted to control the ultrasound transducer array in an imaging mode and in a treatment mode, wherein:
in the imaging mode, an image is acquired capturing the oral geometry of a user of the system, and the controller is adapted to identify a treatment location or locations from the image; and
in the treatment mode, the controller is adapted to control the ultrasound transducer array to deliver ultrasound energy selectively to the identified treatment location or locations.

2. The system of claim 1, wherein the ultrasound transducer array (12) comprises an array of CMUT transducer elements (13).

3. The system of claim 2, wherein the transducer elements (13) are operated in a collapse mode of operation during the treatment mode.

4. The system of any one of claims 1 to 3, wherein the ultrasound patch (12) comprises a flexible printed circuit board carrying the ultrasound transducer array.

5. The system of any one of claims 1 to 4, comprising a multiplexer (16) for multiplexing signals between the controller and the ultrasound patch.

6. The system of any one of claims 1 to 5 for delivering ultrasound energy to regions of dental bone tissue.

7. The system of any one of claims 1 to 6, wherein:
the ultrasound energy used in the treatment mode has a frequency in the range 1MHz to 3MHz; and/or.
the ultrasound energy used in the imaging mode has a frequency in the range 3MHz to 10MHz.

8. The system of any one of claims 1 to 7, wherein in the treatment mode, the transducer array is adapted to provide pulsed ultrasound energy.

9. The system of claim 8, wherein the pulsed ultrasound energy has one or more of:
a pulse repetition frequency in the range 50Hz to 2kHz;
a number of pulses per treatment session in the range 100 to 10,000;
a duty cycle in the range 5% to 50%; and
a time averaged delivered intensity in the range 10mW/cm² to 500mW/cm².

10. The system of any one of claims 1 to 9, wherein the controller (14) is adapted to identify a treatment location or locations from the image by:
identifying landmarks within the image; and
interpolating a treatment location from the landmarks.

11. The system of any one of claims 1 to 10, wherein the controller (14) is adapted to identify a treatment location or locations in real time and the treatment mode is adapted in real time.

12. The system of any one of claims 1 to 11, wherein the controller (14) is adapted to control the ultrasound transducer array to deliver ultrasound energy selectively to the identified treatment location or locations by one or more of:
angular repositioning of the ultrasound probe;
implementing bi-plane imaging; and
activating different parts of the ultrasound array.

13. A computer program comprising computer program code which is adapted, when run on the processor of a controller of a system for providing oral tissue treatment, to cause the system to perform a method comprising:
in an imaging mode:
controlling an array of ultrasound transducers of an ultrasound patch to acquire an image capturing the oral geometry of a user of the system; and
identifying a treatment location or locations from the image; and
in a treatment mode:
controlling the ultrasound transducer array to deliver ultrasound energy selectively to the identified treatment location or locations.

14. The computer program of claim 13, wherein the method comprises, in the treatment mode, providing pulsed ultrasound energy, wherein the pulsed ultrasound energy has one or more of:
a pulse repetition frequency in the range 50Hz to 2kHz;
a number of pulses per treatment session in the range 100 to 10,000;
a duty cycle in the range 5% to 50%;
a time averaged intensity in the range 10mW/cm² to 500mW/cm².

15. A processor having a memory on which is stored the computer program of claim 13 or 14.
